(19) Europäisches Patentamt
European Patent Office
Office européen des brevets

(11) Veröffentlichungsnummer: **0 123 977**
**A1**

# (12) EUROPÄISCHE PATENTANMELDUNG

(21) Anmeldenummer: **84104042.1**

(22) Anmeldetag: **11.04.84**

(51) Int. Cl.³: **C 07 D 295/18**, C 07 D 295/20, C 07 D 307/68, C 07 D 213/82, C 07 D 207/16, C 07 D 409/12, A 61 K 31/495

(30) Priorität: **28.04.83 DE 3315424**

(43) Veröffentlichungstag der Anmeldung: **07.11.84 Patentblatt 84/45**

(84) Benannte Vertragsstaaten: **AT BE CH DE FR GB IT LI NL SE**

(71) Anmelder: **CASSELLA Aktiengesellschaft, Hanauer Landstrasse 526, D-6000 Frankfurt am Main 61 (DE)**

(72) Erfinder: **Schönafinger, Karl, Dr., Holunderweg 8, D-8755 Alzenau (DE)**
Erfinder: **Beyerle, Rudi,Dr., An der Pfaffenmauer 44, D-6000 Frankfurt am Main 60 (DE)**
Erfinder: **Schindler, Ursula, Dr., Reinhardswaldweg 1, D-6082 Mörfelden-Walldorf (DE)**
Erfinder: **Martorana, Piero, Dr., Kaiser-Friedrich-Promenade 108, D-6380 Bad Homburg (DE)**
Erfinder: **Nitz, Rolf-Eberhard, Dr., Heinrich-Bingemer-Weg 64, D-6000 Frankfurt am Main 60 (DE)**

(74) Vertreter: **Urbach, Hans-Georg, Dr. et al, Hanauer Landstrasse 526, D-6000 Frankfurt am Main 61 (DE)**

(54) **Substituierte Piperazin-1-yl-essigsäure-amide, Verfahren zu ihrer Herstellung und ihre Verwendung.**

(57) Substituierte Piperazin-1-yl-essigsäure-amide der Formel I

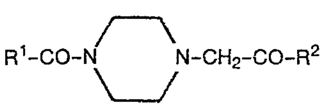

(I)

worin $R^1$ z.B. Phenyl, Pyridyl, Thienyl, Furyl, Amino, Alkylamino, Phenylamino, oder Alkoxy,
$R^2$ z.B. Amino, Alkylamino, Dialkylamino, Morpholino, 4-Methyl-piperazinyl-(1), Pyrrolidinyl-(1), Piperidino, Diethanolamino, Dipropanolamino, $-NH(CH_2)_n-R^3$, $-NH(CH_2)_m-R^4$,
$R^3$ Hydroxy, Alkoxy, Amino, Alkylamino, Dialkylamino,
$R^4$ Phenyl, substituiertes Phenyl, Pyridyl,
n = 2 oder 3,
m = 1 oder 2
bedeuten, besitzen encephalotrope Wirkungen und werden als pharmakologische Wirkstoffe zur Bekämpfung der cerebralen Insuffizienz oder bei der Verbesserung der intellektuellen Leistungsfähigkeit verwendet.

EP 0 123 977 A1

Ref. 3274

Dr.Eu/Ll

**Substituierte Piperazin-1-yl-essigsäure-amide, Verfahren zu ihrer Herstellung und ihre Verwendung**

Die Erfindung betrifft neue substituierte Piperazin-1-yl-essigsäure-amide der Formel I

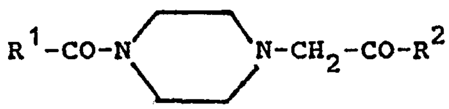

(I)

worin

$R^1$ Phenyl, ein-, zwei- oder dreifach, unabhängig voneinander durch Alkyl($C_1$-$C_4$), -O-Alkyl($C_1$-$C_4$), -CO-O-Alkyl-($C_1$-$C_4$), $-SCH_3$, $-NH_2$, -NH-Alkyl($C_1$-$C_3$), -N(Alkyl)$_2$($C_1$-$C_2$ in jedem Alkylrest), -F, -Cl, -Br, -I, -OH oder -SH substituiertes Phenyl, Pyridyl, Thienyl, Furyl, p-Chlorphenoxymethyl, Amino, Alkylamino mit 1 bis 5 C-Atomen, Phenylamino, im Phenylkern durch -Cl, -Br, $-CH_3$ oder $-OCH_3$ substituiertes Phenylamino oder Alkoxy mit 1 bis 4 C-Atomen,

$R^2$ Amino, Alkylamino mit 1 bis 4 C-Atomen, Dialkylamino mit 1 bis 4 C-Atomen in jedem Alkylrest, Morpholino, 4-Methyl-piperazinyl-(1), Pyrrolidinyl-(1), Piperidino, Cyclohexamethylenimino, Diethanolamino, Dipropanolamino, $-NH(CH_2)_n-R^3$, $-NH(CH_2)_m-R^4$ oder 2-($R^3$-Carbonyl)-pyrrolidin-1-yl,

$R^3$ Hydroxy, Alkoxy mit 1 bis 4 C-Atomen, Amino, Alkylamino mit 1 bis 4 C-Atomen, Dialkylamino mit 1 bis 4 C-Atomen in jedem Alkylrest,

$R^4$ Phenyl, Methoxyphenyl, Methylphenyl, Dimethoxyphenyl, Dimethylphenyl, Pyridyl,

n 2 oder 3,

m 1 oder 2

bedeuten sowie ihre Säureadditionsverbindungen.

Die Erfindung betrifft auch ein Verfahren zur Herstellung der Verbindungen der Formel I und ihre Verwendung als Arzneimittel.

Die für $R^1$, $R^2$, $R^3$ stehenden Alkyl- oder Alkoxyreste können, auch wenn sie als Substituenten anderer Reste auftreten, geradkettig oder verzweigt sein.

Der Rest $R^1$ bedeutet bevorzugt Phenyl, Methoxyphenyl, Chlorphenyl, Bromphenyl, Fluorphenyl, Alkyl($C_1$-$C_4$)-phenyl, 4-Chlorphenoxymethyl, Dimethoxyphenyl, Dichlorophenyl, Methoxycarbonyl-phenyl, Acetoxy-methoxy-phenyl, Alkoxy-($C_1$-$C_2$), Amino, Chlorphenylamino, Trimethoxyphenyl, Thienyl, Pyridyl, Furyl.

Vorgenannte, für $R^1$ stehende bevorzugte substituierte Phenylreste sind dabei vorzugsweise in 3- oder insbesondere 4-Stellung monosubstituiert oder vorzugsweise in 3,4-Stellung disubstituiert oder vorzugsweise in 3,4,5-Stellung trisubstituiert. Ein Pyridyl-rest ist vorzugsweise ein 3-Pyridylrest. Ein Furylrest ist vorzugsweise ein 2-Furylrest. Ein Thienyl-rest ist vorzugsweise ein 2-Thienylrest.
Der Rest $R^2$ bedeutet bevorzugt Amino, Alkyl($C_1$-$C_4$)-amino, Di-alkyl($C_1$-$C_4$)-amino, Morpholino, 4-Methyl-piperazinyl-(1), Pyrrolidinyl-(1), Piperidino, Diethanolamino, $-NH(CH_2)_n-R^3$, $-NH(CH_2)_m-R^4$ oder 2-($R^3$-Carbonyl)-pyrrolidin-1-yl, wobei $R^3$ und $R^4$ vorzugsweise folgende Bedeutungen besitzen:
$R^3$: Hydroxy, Alkoxy mit 1 oder 2 C-Atomen, Amino, Alkylamino mit 1 oder 2 C-Atomen, Dialkylamino mit 1 oder 2 C-Atomen in jedem Alkylrest und
$R^4$: Phenyl, Methoxyphenyl, Dimethoxyphenyl, Pyridyl.

Ganz besonders bevorzugt bedeutet $R^1$ 4-Methoxyphenyl und $R^2$ Morpholino.

Die Verbindungen der Formel I werden dadurch hergestellt, daß
a) ein Piperazin-1-yl-essigsäure-amid der Formel II

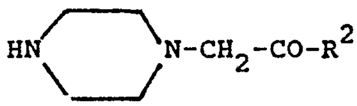 (II)

mit einem Acylierungsmittel III umgesetzt wird, das den Acylrest $R^1$-CO- in die Verbindung II einführt. Für den Fall, daß der Rest $R^1$ über ein Kohlenstoffatom an die Carbonylgruppe der Verbindung I gebunden ist, sind geeignete Acylierungsmittel: Carbonsäurehalogenide, insbesondere Carbonsäurechloride, Carbonsäureanhydride, Carbonsäureester und Carbonsäuren der Formel IIIa

$R^1$-CO-X (IIIa)

worin X = Halogen, -O-CO-$R^1$, -O-Alkyl, -OH bedeutet. Für den Fall, daß der Rest $R^1$ über ein Stickstoffatom an die Carbonylgruppe der Verbindung I gebunden ist, sind Isocyansäureester bzw. Isocyansäure der Formel IIIb

$R^5$-N=C=O (IIIb)

geeignete Acylierungsmittel. In der Formel IIIb wird die Bedeutung des Restes $R^5$ so gewählt, daß der Rest: ($R^5$-NH-) mit dem für $R^1$ stehenden Rest: Amino, Alkylamino mit 1 bis 5 C-Atomen, Phenylamino, im Phenylkern durch -Cl, -Br, $-CH_3$ oder $-OCH_3$ substituiertes Phenylamino identisch ist. $R^5$ kann demnach bedeuten: Wasserstoff, Alkyl($C_1$-$C_5$), Phenyl, durch -Cl, -Br, $-CH_3$ oder $-OCH_3$ substituiertes Phenyl.
Für den Fall, daß der Rest $R^1$ über ein Sauerstoffatom an die Carbonylgruppe der Verbindung I gebunden ist, sind z.B. Chlorameisensäureester der Formel IIIc

Alkyl($C_1$-$C_4$)-O-CO-Cl (IIIc)

als Acylierungsmittel geeignet. Oder daß
b) ein Piperazid der Formel IV mit einer Verbindung der Formel V

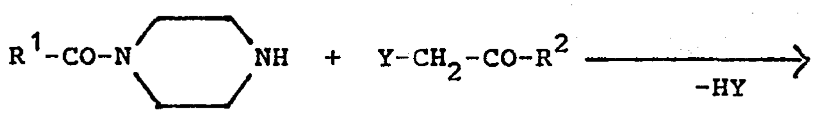

(I)

(IV) (V)

umgesetzt wird, wobei Y Halogen, insbesondere Cl, $-OSO_2CH_3$, $-OSO_2$-Phenyl oder o-Tosyl bedeutet, oder daß

c) ein Piperazin-1-yl-essigsäure-derivat der Formel VI mit einem Amin der Formel VII

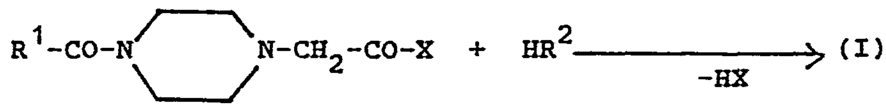

(VI) (VII)

umgesetzt wird, wobei X die bei dem Verfahren a) angegebenen Bedeutungen besitzt.

Vorzugsweise bedeutet X bei den Verfahren a) und c) Cl oder O-Alkyl($C_1$-$C_4$). In den Verbindungen II bis VI haben die Reste $R^1$ und $R^2$ die bereits eingangs erwähnten Bedeutungen.

Die Verfahren a) bis c) sind gängige chemische Verfahrensschritte. Vorzugsweise werden die Verfahren a) bis c) in einem geeigneten inerten Lösungsmittel durchgeführt. Geeignete Lösungsmittel sind z.B. Alkohole, vorzugsweise für die Reaktion b), insbesondere solche mit 1 bis 6 C-Atomen, wie z.B. Methanol, Ethanol, i-und n-Propanol, i-, sec- und tert-Butanol, n-, i-, sec-, tert-Pentanol, n-Hexanol, Cyclopentanol, Cyclohexanol; Ether, insbesondere solche mit 2 bis 8 C-Atomen im Molekül, wie z.B. Diethylether, Methylethylether, Di-n-propyl-ether, Di-iso-propylether, Methyl-n-butyl-ether, Ethylpropylether, Di-n-butyl-ether, Tetrahydrofuran, 1,4-Dioxan, 1,2-Dimethoxyethan, Bis-ß-methoxyethylether; Polyether, wie z.B. Polyethylenglykole mit einem Molekulargewicht bis ca. 600; Oligoethylen-glycol-dimethylether, wie z.B. Pentaglyme; Glykole und teilweise veretherte Glykole, wie z.B. Ethylenglykol, Propylenglykol, Trimethylenglykol, Ethylenglykol-monomethyl-ether, Ethylenglykol-monoethylether, Diethylenglykol-mono-ethyl-ether; Ketone, insbesondere

solche mit 3 bis 10 C-Atomen im Molekül, wie z.B. Aceton, Methylethylketon, Methyl-n-propylketon, Diethylketon, 2-Hexanon, 3-Hexanon, Di-n-propylketon, Di-iso-propylketon, Di-iso-butylketon, Cyclopentanon, Cyclohexanon, Benzophenon, Acetophenon; aliphatische Kohlenwasserstoffe, wie z.B. niedrig-und hochsiedende Petrol-ether; aromatische Kohlenwasserstoffe, wie z.B. Benzol, Toluol, o-, m- und p-Xylol, Pyridin; halogenierte aliphatische oder aromatische Kohlenwasserstoffe, wie z.B. Methylenchlorid, Chloroform, Tetrachlorkohlenstoff, Ethylenchlorid, Chlorbenzol, Dichlorbenzol; Nitrile, wie z.B. Acetonitril; Amide, wie z.B. Dimethylformamid, N-Methyl-pyrrolidon; Hexamethylphosphorsäuretriamid; Sulfoxide, wie z.B. Dimethylsulfoxid; Wasser. Auch Gemische verschiedener Lösungsmittel können verwendet werden.

Die Umsetzung wird bei allen Verfahrensschritten in der Regel bei Raumtemperatur durchgeführt. Je nach der Reaktivität der Reaktanten kann es aber günstig sein, die Reaktion unter Kühlung bei tieferen Temperaturen oder bei erhöhter Temperatur, z.B. bis zur Rückflußtemperatur des verwendeten Lösungsmittels oder Lösungsmittelgemischs, durchzuführen. Die Reaktionstemperatur liegt in vielen Fällen bei $-10^{o}C$ bis $25^{o}C$, vorzugsweise bei $0^{o}C$ bis $20^{o}C$.

Hinsichtlich einer hohen Reaktionsgeschwindigkeit und hoher Ausbeute ist es in der Regel auch vorteilhaft, bei den Reaktionen a) bis c), bei denen als HX oder HY Chlorwasserstoff abgespalten wird, als Säurefänger zusätzlich eine Base einzusetzen. Derartige geeignete Basen sind z.B.: Alkalicarbonate, wie Soda oder Pottasche, Alkalibikarbonate, wie $NaHCO_3$ oder organische Amine, insbesondere tertiäre organische Amine wie Pyridin, oder tertiäre aliphatische Amine mit 3 bis 9 C-Atomen, wie Trimethylamin, Triethylamin, Tri-n-propylamin.

Falls bei den Reaktionen a) und c) Wasser abgespalten wird, ist es günstig, die Reaktion im wasserfreien Medium durchzuführen und zweckmäßigerweise einen Wasserfänger zuzusetzen.

Geeignete Wasserfänger sind z.B. Carbodiimide, wie Dicyclohexylcarbodiimid.

Bei der Herstellung der Verbindungen der Formel I nach den Verfahrensschritten a) bis c) werden die Ausgangskomponenten normalerweise in etwa äquimolaren Mengen eingesetzt. Falls ein Ausgangsamin auch als Säurefänger wirken soll, wird das Amin im Überschuß eingesetzt. Dieser Überschuß kann z.B. bis zu 10 molar oder noch größer sein. Die Aufarbeitung der Ansätze erfolgt nach üblichen Verfahren.

Die zur Herstellung der Verbindungen der Formel I benötigten Ausgangsprodukte der Formeln II bis VII sind bekannt oder können leicht nach den für die jeweilige Verbindungsklasse bekannten Verfahren hergestellt werden.

Geeignete Ausgangsverbindungen der Formel II sind z.B. (Piperazin-1-yl-essigsäure)-amid, -methylamid, -ethylamid, -isopropylamid, -butylamid, -dimethylamid, -diethylamid, -benzylamid, -N-methylbenzylamid, -morpholid, -pyrrolidid, -piperidid, -N-methyl-piperazid, -cyclohexamethylenimid, -diethanolamid, -(2-methoxy)-ethylamid, -(2-ethoxy)-ethylamid, -dipropanolamid, -(3-methoxy)-propylamid, -2-methoxy-carbonyl-pyrrolidid, -2-aminocarbonyl-pyrrolidid, -diethylaminoethylamid, -dimethylaminopropylamid, -methylaminoethylamid, -3,4-dimethoxyphenylethylamid, -4-methoxy-benzylamid, -pyrid-3-yl-methylamid, -pyrid-2-yl-methylamid. Die Ausgangsverbindungen der Formel II können, sofern sie nicht bereits bekannt sind, z.B. durch Umsetzung von Piperazin mit einer Verbindung der Formel V, oder durch Umsetzung eines Piperazin-1-yl-essigsäure-alkylesters der Formel VIII

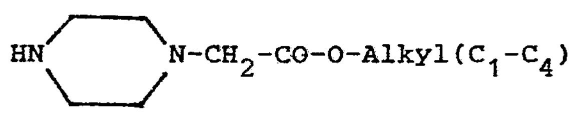 (VIII)

mit einem Amin der Formel VII hergestellt werden. Diese Umsetzungen werden vorzugsweise in einem der bereits genannten Lösungsmittel durchgeführt.

Geeignete Acylierungsmittel der Formel III sind zum Beispiel: Benzoylchlorid, 2-, 3- oder 4-Methoxybenzoylchlorid, 2,3-, 2,4- oder 3,4-Dimethoxybenzoylchlorid, 2-, 3- oder 4-Chlorbenzoylchlorid, Nikotinsäuremethylester, 2- und 3-Thenoylchlorid, 2- und 3-Furoylchlorid, 4-Chlorphenoxyacetylchlorid, 2-, 3- oder 4-Chlorphenylisocyanat, Isocyansäure (Kaliumcyanat + Mineralsäure), Benzoesäure, Benzoesäuremethylester, Benzoesäureanhydrid, Isonicotinsäurechlorid, 4-Hydroxy-3-methoxy-benzoesäuremethylester, 3,4,5-Trimethoxybenzoylchlorid, 3,4-Dichlorbenzoylchlorid, 2-, 3-, 4-Fluorbenzoylchlorid, 2-, 3-, 4-Brombenzoylchlorid, 4-tert-Butylbenzoylchlorid, 4-Methoxycarbonyl-benzoylchlorid, 4-Dimethylamino-benzoesäurechlorid, 2-, 3-, 4-Methylmercaptobenzoesäuremethylester, Methylisocyanat, Isopropylisocyanat, Chlorameisensäure-methyl-, -ethyl-, -n-propyl-, -i-propyl-, -n-butyl-, -i-butyl-, -tert-butyl-ester. Die Acylierungsmittel der Formel III können leicht nach den für die Herstellung von Säurehalogeniden, insbesondere Säurechloriden, Carbonsäureestern, Carbonsäureanhydriden, Isocyanaten und Chlorameisensäureestern bekannten Verfahren synthetisiert werden.

Geeignete Piperazide der Formel IV können leicht durch Umsetzung von Piperazin mit einem Acylierungsmittel IIIa, IIIb und IIIc nach bekannten Verfahren hergestellt werden. Geeignete Piperazide der Formel IV sind z.B.: Benzoesäure-, 2-, 3-, oder 4-Methoxybenzoesäure-, 2,3- oder 3,4-Dimethoxy-benzoesäure-, 2-, 3- oder 4-Chlorbenzoesäure-, Nicotinsäure-, 2- und 3-Thiophencarbonsäure-, 2- und 3-Furancarbonsäure-, 4-Chlorphenoxyessigsäure-, Isonicotinsäure-, 3,4,5-Trimethoxybenzoesäure-, 3,4-Dichlorbenzoesäure-, 2-, 3-, 4-Fluorbenzoesäure-, 2-, 3-, 4-Brombenzoesäure-, 2-, 3-, 4-Chlorbenzoesäure-, 4-tert-Butylbenzoesäure-, 4-Methoxycarbonylbenzoesäure-, 4-Dimethylamino-benzoesäure-, 2-, 3-, 4-Methylmercapto-benzoesäure-piperazid; N-(Aminocarbonyl)-piperazin, N-(Methylaminocarbonyl)-piperazin, N-(Ethylaminocar-

bonyl)-piperazin, N-(Isopropylaminocarbonyl)-piperazin, N-(tert.Butylaminocarbonyl)-piperazin, N-(n-Butylaminocarbonyl)-piperazin, N-(n-Pentylaminocarbonyl)-piperazin, N-(Isopentylaminocarbonyl)-piperazin, N-(Methoxycarbonyl)-piperazin, N-(Ethoxycarbonyl)-piperazin, N-(Isopropoxycarbonyl)-piperazin, N-(n-Propoxycarbonyl)-piperazin, N-(n-Butoxycarbonyl)-piperazin, N-(Isobutoxycarbonyl)-piperazin.

Geeignete Ausgangsverbindungen der Formel V sind z.B.: (Chlor-, Brom-, Mesyloxy-, Phenylsulfonyloxy- oder p-Tosyloxy-, -essigsäure)-methylamid, -ethylamid, -n-propylamid, -isopropylamid, -n-butylamid, -isobutylamid, -dimethylamid, -diethylamid, -di-n-propylamid, -di-n-butylamid, -N-methyl-N-n-propylamid, -benzylamid, -methylbenzylamid, -morpholid, -pyrrolidid, -piperidid, -N-methyl-piperazid, -cyclohexamethylenimid, -diethanolamid, -2-(methoxy)-ethylamid, -2-(ethoxy)-ethylamid, -dipropanolamid, -3-(methoxy)-propylamid, -2-(methoxy-carbonyl)-pyrrolidid, -2-aminocarbonyl-pyrrolidid, -diethylaminoethylamid, -dimethylaminopropylamid, -methylaminoethylamid, -3,4-dimethoxyphenylethylamid, -4-methoxybenzylamid, -pyrid-3-yl-methylamid, -pyrid-2-yl-methylamid. Die Ausgangsverbindungen der Formel V können z.B. hergestellt werden durch Acylierung eines Amins der Formel VII mit Chloracetylchlorid nach bekannten Verfahren.

Geeignete Ausgangsverbindungen der Formel VI sind z.B. : (4-Benzoyl-piperazin-1-yl)-essigsäure-methyl- oder -ethylester, (4-(4-Methoxybenzoyl)-piperazin-1-yl)-essigsäure-methyl- oder -ethyl-ester, (4-(2,3-, 2,4- oder 3,4-Dimethoxybenzoyl)-piperazin-1-yl)-essigsäure-methyl- oder -ethylester, (4-(2-, 3- oder 4-Chlorbenzoyl)-piperazin-1-yl)-essigsäure-methyl-oder -ethyl-ester, (4-Nicotinoyl-piperazin-1-yl)-essigsäure-methyl- oder -ethyl-ester, (4-(2- oder 3-Furoyl)-piperazin-1-yl)-essigsäure-methyl- oder -ethyl-ester, (4-(2- oder 3-Thenoyl)-piperazin-1-yl)-essigsäure-methyl-oder -ethyl-ester, (4-Aminocarbonyl-piperazin-1-yl)-essigsäure-methyl- oder ethyl-ester, (4-Isopropylaminocarbonyl-piperazin-1-yl)-essigsäure-methyl- oder ethyl-ester, (4-Phenylamino-carbonyl-piperazin-1-yl)-essigsäure-methyl- oder -ethyl-ester, (4-(4-Methoxyphenylamino-carbonyl)-piperazin-1-yl)-essigsäure-methyl- oder -ethyl-ester, (4-(2-, 3- oder 4-Fluorbenzoyl)-piperazin-1-yl)-acetyl-chlorid, (4-(2-, 3- oder 4-Chlorbenzoyl)-piperazin-1-yl)-acetyl-chlorid, (4-(2-, 3- oder 4-Dimethylaminobenzoyl)-piperazin-1-yl)-acetyl-chlorid, (4-(2-, 3- oder 4-Methylmercapto)-piperazin-1-yl)-acetyl-chlorid.

Die Ausgangsverbindungen der Formel VI können z.B. durch Umsetzung einer Verbindung IV mit einer Verbindung der Formel $Hal-CH_2-CO-Alkyl(C_1-C_4)$, worin Hal Halogen, insbesondere Chlor, bedeutet, oder durch Umsetzung eines Piperazin-1-yl-essigsäure-alkylesters der Formel VIII mit einem Acylierungsmittel IIIa, IIIb oder IIIc hergestellt werden.

Die als Ausgangsprodukte benötigten Amine der Formel VII sind bekannt oder können leicht nach literaturbekannten Verfahren hergestellt werden. Beispiele für Amine der Formel VII sind: Ammoniak, Methylamin, Ethylamin, n-Propylamin, i-Propylamin, n-Butylamin, sec-Butylamin, i-Butylamin, 2-(Ethoxy)-ethylamin, 2-(Methoxy)-ethylamin, Benzylamin, 2-Phenethyl-amin, Morpholin, N-Methylpiperazin, Pyrrolidin, Piperidin, Diethylamin, Di-n-propylamin, Di-n-butylamin, N-Methyl-N-ethyl-amin, N-Methyl-N-n-butyl-amin, 2-(Hydroxycarbonyl)-pyrrolidin, 2-(Methoxy-carbonyl)-pyrrolidin, 2-(Ethoxy-carbonyl)-pyrrolidin, 2-(n-Propoxy-carbonyl)-pyrrolidin, 2-(tert.-Butoxy-carbonyl)-pyrrolidin, 2-(Amino-carbonyl)-pyrrolidin, 2-(Methylamino-carbonyl)-pyrrolidin, 2-(n-Butylamino-carbonyl)-pyrrolidin, 2-(Dimethylamino-carbonyl)-pyrrolidin, 2-(Dibutylamino-carbonyl)-pyrrolidin, 2-(2-, 3- oder 4-Methoxy)-phenethyl-amin, 2-(2-, 3- oder 4-Methyl)-phenethyl-amin, 2-(2-, 3- oder 4-Pyridyl)-ethyl-amin, 2-, 3-oder 4-Pyridyl-methylamin, 3-(Diethylamino)-propyl-amin.

Die substituierten Piperazin-1-yl-essigsäure-amide der Formel I bilden mit anorganischen oder organischen Säuren Säureadditionssalze. Zur Bildung derartiger Säureadditionssalze sind anorganische und organische Säuren geeignet. Geeignete Säuren sind beispielsweise:
Chlorwasserstoff, Bromwasserstoff, Naphthalindisulfonsäuren, insbesondere Naphthalin-1,5-disulfonsäure, Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Nicotin-, Isonicotin-, Methansulfon-, p-Toluolsulfon-, Zitronen- oder Adipin-Säure. Pharmakologisch annehmbare Säureadditionssalze werden bevorzugt. Die Säureadditionssalze werden wie üblich durch Vereinigung der Komponenten, zweckmäßigerweise in einem geeigneten Lösungs-oder Verdünnungsmittel, hergestellt. Bei der Synthese der Verbindungen der Formel I können zunächst die Säureadditionssalze im Zuge der Aufarbeitung anfallen. Aus den Säureadditionssalzen können die freien Verbindungen der allgemeinen Formel I gewünschtenfalls in bekannter Weise, z.B. durch Auflösen oder Suspendieren in Wasser und Alkalischstellen, z.B. mit Natronlauge, und anschließendes Filtrieren, gewonnen werden.

Die erfindungsgemäßen Verbindungen der Formel I und ihre pharmakologisch annehmbaren Säureadditionssalze besitzen wertvolle pharmakologische Eigenschaften. Aufgrund ihrer encephalotropen Wirkung können sie als Mittel zur Verbesserung zerebraler Funktionen, z.B. von Gedächtnis und Lernfähigkeit, eingesetzt werden. Sie stellen somit eine Bereicherung der Pharmazie dar und lassen sich zur Behandlung und Vorbeugung menschlicher Krankheiten, insbesondere bei der Bekämpfung und Verhütung der cerebralen Insuffizienz und bei der Verbesserung der intellektuellen Leistungsfähigkeit einsetzen.

Die Verbindungen der Formel I und ihre pharmakologisch annehmbaren Säureadditionssalze können daher am Menschen als Heilmittel für sich allein, in Mischungen untereinander oder

in Form von pharmazeutischen Zubereitungen verabreicht werden, die eine enterale oder parenterale Anwendung gestatten und die als aktiven Bestandteil eine wirksame Dosis mindestens einer Verbindung der Formel I oder eines Säureadditionssalzes davon, neben üblichen pharmazeutisch einwandfreien Träger-und Zusatzstoffen enthalten. Die Zubereitungen enthalten normalerweise etwa 0,5 bis 90 Gewichtsprozent der therapeutisch wirksamen Verbindung.

Die Heilmittel können oral, z.B. in Form von Pillen, Tabletten, Lacktabletten, Dragees, Granulaten, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Die Herstellung der pharmazeutischen Präparate erfolgt in an sich bekannter Weise, wobei pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glycerin, Polyole, pflanzliche Öle etc.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel I oder ihrer pharmakologisch annehmbaren Säureadditionssalze und noch andere therapeutisch wirksame Stoffe enthalten.

Derartige andere therapeutisch wirksame Substanzen sind beispielsweise: ß-Rezeptorenblocker, wie z.B. Propranolol, Pindolol, Metoprolol; antianginöse Mittel wie z.B. Carbochromen; Molsidomin; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Prazosin; Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon.

Die Verbindungen der Formel I, ihre pharmakologisch annehmbaren Säureadditionssalze und pharmazeutische Präparate, welche die Verbindungen der Formel I oder ihre pharmakologisch annehmbaren Säureadditionssalze als Wirkstoff enthalten, können am Menschen bei der Bekämpfung bzw. Vorbeugung von Erkrankungen verwendet werden, insbesondere bei der Bekämpfung bzw. Vorbeugung der cerebralen Insuffizienz und bei der Verbesserung der intellektuellen Leistungsfähigkeit. Die Dosierung kann innerhalb weiter Grenzen variieren und ist in jedem einzelnen Fall den individuellen Gegebenheiten anzupassen. Im allgemeinen ist bei oraler Verabreichung eine Tagesdosis von etwa 1 bis 1000 mg/kg, vorzugsweise 5 bis 800 mg/kg, Körpergewicht zur Erzielung wirksamer Ergebnisse angemessen, bei intravenöser Applikation beträgt die Tagesdosis im allgemeinen etwa 5 bis 500 mg/kg, vorzugsweise 5 bis 250 mg/kg, Körpergewicht. Die

Tagesdosis wird normalerweise, insbesondere bei der Applikation größerer Mengen, in mehrere, z.B. 2, 3 oder 4, Teilverabreichungen aufgeteilt. Gegebenenfalls kann es, je nach individuellem Verhalten, erforderlich werden, von der angegebenen Tagesdosis nach oben oder unten abzuweichen.

Die Prüfung der erfindungsgemäßen Verbindungen auf encephalotrope Wirkung erfolgte z.B. durch den passive avoidance Test, der wie folgt durchgeführt wird:
Die Testapparatur ist eine Hell-Dunkel-Box mit elektrifizierbarem Gitterboden im dunklen Teil.

90 Minuten nach Verabreichung von Kontroll- und Präparatinjektion werden unerfahrene männliche Mäuse mit Scopolaminhydrobromid (3 mg/kg s.c.) behandelt. 5 Minuten später werden die Mäuse in den hellen Teil der Box gesetzt. Nach dem Überwechseln in den dunklen Teil der Box erhalten sie einen ihnen unangenehmen elektrischen Fußschock. Nach 24 Stunden wird jede Maus einmal in den hellen Teil der Testapparatur gesetzt und die Verweildauer (max.180 sec) gemessen. Die signifikante Wirkung der Testsubstanz im Vergleich zur Kontrollgruppe wird mittels Mediantest berechnet.

Als minimale effektive Dosis MED eines Präparats bezeichnet man diejenige, die eine signifikante Wirkung gegen Scopolamin zeigt. Die mit einer aktiven Dosis eines Präparats und Scopolamin behandelten Tiere zeigen eine lange Verweildauer, ebenso die nicht mit Scopolamin behandelten Tiere, wogegen diejenigen mit Kontrollinjektion und Scopolamin behandelten eine kurze Verweildauer zeigen. Zum Vergleich wurde die MED von Piracetam mitbestimmt. Bei der Prüfung werden die in der nachfolgenden Tabelle angegebenen Werte erhalten:

| Verbindung gemäß nachfolgender Beispiele | MED in mg/kg p.o. |
|---|---|
| 1, 3, 5 | 3 |
| 7, 13, 28 | kleiner als 30 |
| 2, 8, 10, 11, 12, 13, 16, 17, 27, 29, 30 | 30 |
| Piracetam (Vergleich) | 100 |

Beispiel 1:

(4-(4-Methoxybenzoyl)-piperazin-1-yl)-essigsäure-morpholid-hydrochlorid.

10,25 g Piperazin-1-yl-essigsäure-morpholid werden in 20 ml Methylenchlorid auf $0^{o}C$ abgekühlt. 8,75 g Anisoylchlorid werden innerhalb von 15 Minuten zugetropft. Nach 4 h Nachrühren wird mit Isopropanol verdünnt, wobei das farblose Produkt ausfällt. Es wird abgesaugt und getrocknet.

Ausbeute: 14,6 g Fp: 176 - $178^{o}C$

Elementaranalyse:

| | | | | | |
|---|---|---|---|---|---|
| Berechnet: | C 56,3 | H 6,8 | Cl 9,3 | N 10,9 | O 16,7 |
| Gefunden: | C 56,1 | H 6,6 | Cl 9,5 | N 10,6 | O 17,0 |

Beispiel 2:

(4-Aminocarbonyl-piperazin-1-yl)-essigsäure-morpholid

9,1 g Piperazin-1-yl-essigsäure-morpholid werden in 30 ml Wasser und 4,4 ml konz. Salzsäure gelöst und auf $20^{o}C$ abgekühlt. Die Lösung von 3,1 g Kaliumcyanat in 15 ml Wasser wird unter Kühlung langsam zugetropft. Danach werden weitere 4,4 ml konz. Salzsäure zugegeben und über Nacht bei $20^{o}C$ stehen gelassen. Die Lösung wird mit 2 N Natronlauge neutral gestellt und eingeengt. Auskochen des festen Rückstands mit

Isopropanol und Abkühlen der Lösung liefert 5,3 g farblose Kristalle, die aus Ethanol umkristallisiert werden.
Fp: 165 $^{\circ}$C.
Elementaranalyse:

| | | | | |
|---|---|---|---|---|
| Berechnet: | C 51,6 | H 7,8 | N 21,9 | O 18,8 |
| Gefunden: | C 51,3 | H 7,6 | N 22,7 | O 18,6 |

Beispiel 3:

(4-(4-Methoxybenzoyl)-piperazin-1-yl)-essigsäure-pyrrolidid-hydrochlorid

9,4 g Piperazin-1-yl-essigsäure-pyrrolidid werden in 10 ml Methylenchlorid gelöst. Bei 0 $^{\circ}$C wird die Lösung von 8,75 g Anissäurechlorid in 10 ml Methylenchlorid zugetropft. Nach 60 Minuten Rühren bei 25 $^{\circ}$C werden die Kristalle abgesaugt und im Vakuum getrocknet.
Ausbeute: 10,7 g Fp: 245 $^{\circ}$C
Elementaranalyse:

| | | | | | |
|---|---|---|---|---|---|
| Berechnet: | C 58,8 | H 7,1 | N 11,4 | O 13,1 | Cl 9,7 |
| Gefunden: | C 58,5 | H 7,0 | N 11,1 | O 13,5 | Cl 10,0 |

Beispiel 4:

(4-(4-Methoxybenzoyl)-piperazin-1-yl)-essigsäure-(2-aminocarbonyl)-pyrrolidid

a) 6 g Chloressigsäure-(2-(aminocarbonyl)-pyrrolidid), 7 g (4-Anisoyl)-piperazin und 4,4 g Pottasche werden 2 h in 50 ml Dimethylformamid bei 50 $^{\circ}$C gerührt. Die Reaktionsmischung wird eingeengt und der Rückstand in 100 ml Wasser aufgenommen. Die wäßrige Lösung wird mit Pottasche versetzt und mit Methylenchlorid mehrmals ausgeschüttelt. Die organische Phase ergibt nach dem Trocknen und Einengen einen öligen Rückstand, der beim Verreiben mit Essigester fest wird. Er wird abgesaugt und mit Essigester nachgewaschen.
Fp.: 149 - 150 $^{\circ}$C.

Analyse:

| | | | | |
|---|---|---|---|---|
| Berechnet: | C 61,0 | H 7,0 | N 15,0 | O 17,1 |
| Gefunden: | C 60,9 | H 7,0 | N 15,2 | O 17,0 |

b) Das als Ausgangsprodukt benötigte Chloressigsäure-(2-(aminocarbonyl)-pyrrolidid) wird wie folgt hergestellt:

11,4 g Prolinamid und 10,1 g Triethylamin werden in 100 ml Methylenchlorid gelöst. Bei 30°C werden 11,3 g Chloracetylchlorid zugetropft und anschließend 6 h bei Raumtemperatur gerührt. Nach dem Einengen im Vakuum wird der Rückstand in 150 ml Wasser gelöst und die erhaltene Mischung mit 2 x 100 ml Methylenchlorid ausgeschüttelt. Die organische Phase wird über $Na_2SO_4$ getrocknet und das Methylenchlorid am Rotationsverdampfer entfernt. Der Rückstand wird aus Isopropanol umkristallisiert.
Fp.: 136 - 137°C.
Analyse:

| | | | | | |
|---|---|---|---|---|---|
| Berechnet: | C 44,1 | H 5,8 | Cl 18,6 | N 14,7 | O 16,8 |
| Gefunden: | C 44,0 | H 5,6 | Cl 18,5 | N 15,0 | O 16,8 |

Beispiel 5:

(4-(4-Methoxybenzoyl)-piperazin-1-yl)-essigsäure-morpholid

Zur Mischung von 22 g N-(4-Methoxybenzoyl)-piperazin, 15,2 g Pottasche und 200 ml Toluol wird die Lösung von 15,4 g Chloressigsäuremorpholid in 50 ml Toluol bei Raumtemperatur getropft. Dann wird 6 h unter Rühren zum Rückfluß erhitzt und heiß filtriert. Aus dem Filtrat fallen beim Abkühlen farblose Kristalle aus.
Ausbeute: 23,5 g, Fp. 148-149°C
Elementaranalyse:

| | | | | |
|---|---|---|---|---|
| Berechnet: | C 62,2 | H 7,3 | N 12,1 | O 18,4 |
| Gefunden: | C 62,0 | H 7,2 | N 12,2 | O 18,3 |

Das als Ausgangsprodukt eingesetzte N-(4-Methoxybenzoyl)-piperazin wurde durch Umsetzung von 1 Mol Piperazin mit 0,5 Mol Anissäurechlorid in Eisessig als zähes Öl erhalten.
Das als Ausgangsprodukt eingesetzte Chloressigsäuremorpholid wurde durch Umsetzung von 2 Mol Morpholin mit 1 Mol Chloracetylchlorid in Toluol als farbloses Öl erhalten.

Beispiel 6:

(4-(4-Chlorbenzoyl)-piperazin-1-yl)-essigsäure-morpholid-hydrochlorid.

5 g Piperazinoessigsäuremorpholid und 4,2 g Chlorbenzoylchlorid werden bei 0°C in 50 ml Methylenchlorid zusammengegeben. Die Mischung wird 2 h beim Raumtemperatur gerührt und erneut auf 0°C abgekühlt, wobei ein Niederschlag ausfällt, der aus Isopropanol umkristallisiert wird.
Ausbeute: 7,6 g, Fp: 236-238°C

| | | | | | |
|---|---|---|---|---|---|
| Berechnet: | C 52,5 | H 5,9 | N 10,8 | O 12,4 | Cl 18,3 |
| Gefunden: | C 52,5 | H 5,9 | N 10,6 | O 12,6 | Cl 18,6 |

Beispiel 7:

(4-(4-Methoxybenzoyl)-piperazin-1-yl)-essigsäure-isopropylamid-hydrochlorid

5 g Piperazinoessigsäure-isopropylamid werden in 20 ml Methylenchlorid bei 0°C tropfenweise mit einer Lösung von 4,6 g Anissäurechlorid in 20 ml Methylenchlorid versetzt. Die Mischung wird anschließend 4 h bei Raumtemperatur gerührt und dann eingeengt. Der Rückstand wird aus Isopropanol umkristallisiert.
Ausbeute: 7,5 g, Fp: 227-230°C

| | | | | | |
|---|---|---|---|---|---|
| Berechnet: | C 57,4 | H 7,3 | N 11,8 | O 13,5 | Cl 10,0 |
| Gefunden: | C 57,6 | H 7,3 | N 11,9 | O 13,2 | Cl 10,3 |

In ähnlicher Weise, wie in den Beispielen 1 bis 7 beschrieben, lassen sich die folgenden Verbindungen herstellen:

| Beispiel | | Fp: °C |
|---|---|---|
| 8 | (4-Benzoyl-piperazin-1-yl)-essigsäureamid | 204-206 |
| 9 | (4-(4-Methylbenzoyl)-piperazin-1-yl)-essigsäure-methylamid | 178-179 |
| 10 | (4-Nicotinoyl-piperazin-1-yl-)-essigsäure-dibutylamid | 89-91 |
| 11 | (4-(4-Acetoxy-3-methoxy-benzoyl)-piperazin-1-yl)-essigsäure-dimethylamid | 167-168 |
| 12 | (4-(3,4-Dimethoxybenzoyl)-piperazin-1-yl-)-essigsäure-2-methoxyethylamid | 163-164 |
| 13 | (4-(3-Chlorphenylaminocarbonyl)-piperazin-1-yl-)-essigsäure-piperidid | 177-180 |
| 14 | (4-(4-Chlorbenzoyl)-piperazin-1-yl-)-essigsäure-2-(diethylamino)-ethylamid | 102-104 |
| 15 | (4-Ethoxycarbonyl-piperazin-1-yl-)-essigsäure-benzylamid | 131-132 |
| 16 | (4-(4-Chlorphenoxyacetyl)-piperazin-1-yl-)-essigsäure-N-methylpiperazid | 101-102 |
| 17 | (4-(3,4,5-Trimethoxybenzoyl)-piperazin-1-yl-)-essigsäure-2-(3,4-dimethoxy-phenyl)-ethylamid | 201-203 |
| 18 | (4-(3,4-Dichlorbenzoyl)-piperazin-1-yl-)-essigsäure-4-methoxybenzylamid | 199-200 |
| 19 | (4-(4-Methoxybenzoyl)-piperazin-1-yl-)-essigsäure-diethanolamid | Öl |
| 20 | (4-(4-Methoxybenzoyl)-piperazin-1-yl-)-essigsäure-2-(ethylamino-carbonyl)-pyrrolidid | Öl |
| 21 | (4-(4-Fluorbenzoyl)-piperazin-1-yl-)-essigsäure-2-(methylamino-carbonyl)-pyrrolidid | 98-101 |
| 22 | (4-(3-Brombenzoyl)-piperazin-1-yl-)-essigsäure-isopropylamid | 213-215 |
| 23 | (4-(4-tert.-Butylbenzoyl)-piperazin-1-yl-)-essigsäure-isopropylamid | 187-189 |

| | | |
|---|---|---|
| 24 | (4-(4-Methoxycarbonyl-benzoyl)-piperazin-1-yl-)-essigsäure-(2-hydroxyethyl)-amid | 147-149 |
| 25 | (4-(2-Furoyl)-piperazin-1-yl-)-essigsäure-(3-methoxy-propyl)-amid | 150-152 |
| 26 | (4-(4-Chlorbenzoyl)-piperazin-1-yl-)-essigsäure-morpholid-hydrochlorid | 235-238 |
| 27 | (4-(Benzoyl)-piperazin-1-yl-)-essigsäure-morpholid | 135-137 |
| 28 | (4-(3-Chlorphenylamino-carbonyl)-piperazin-1-yl-)-essigsäure-morpholid | 155-157 |
| 29 | (4-(3-Methoxy-4-acetoxy-benzoyl)-piperazin-1-yl-)-essigsäure-morpholid-hydrochlorid | Zers.ab 70°C |
| 30 | (4-(4-Methoxy-benzoyl)-piperazin-1-yl-)-essigsäure-(2-Methoxycarbonyl)-pyrrolidid) | 87-89 |
| 31 | (4-(2-Thenoyl)-piperazin-1-yl-)-essigsäure-(2-pyridyl-methyl)-amid | 135-137 |

In den nachfolgenden Beispielen werden pharmazeutische Präparate beschrieben, wobei anstelle des als Wirkstoff verwendeten (4-(Methoxybenzoyl)-piperazin-1-yl)-essigsäure-morpholid auch ein anderer erfindungsgemäßer Wirkstoff verwendet werden kann.

Beispiel 32:

Gelatineweichkapseln, enthaltend 40 mg Wirkstoff pro Kapsel:

| | pro Kapsel |
|---|---|
| (4-(Methoxybenzoyl)-piperazin-1-yl)-essigsäure-morpholid | 40 mg |
| Aus Kokosfett fraktioniertes Triglyceridgemisch | 150 mg |
| Kapselinhalt | 190 mg |

Beispiel 33:

Injektionslösung, enthaltend 10 mg Wirkstoff pro ml:

| | | pro ml |
|---|---|---|
| (4-(Methoxybenzoyl)-piperazin-1-yl)-essigsäure-morpholid | | 10 mg |
| Natriumchlorid | | 2,7 mg |
| Wasser zu Injektionszwecken | ad | 1,0 ml |

Beispiel 34:

Emulsion, enthaltend 25 mg Wirkstoff pro 5 ml

| | | pro 100 ml |
|---|---|---|
| (4-(Methoxybenzoyl)-piperazin-1-yl)-essigsäure-morpholid | | 0,5 g |
| Neutralöl | | q.s. |
| Polyoxiethylen-stearat | | q.s. |
| Glycerin rein | | 0,2 bis 2,0 g |
| Geschmacksstoff | | q.s. |
| Wasser (entsalzt oder destilliert) | ad | 100 ml |

Beispiel 35:

Rektale Arzneiform, enthaltend 15 mg Wirkstoff pro Suppositorium

| | | pro Suppositorium |
|---|---|---|
| (4-(Methoxybenzoyl)-piperazin-1-yl)-essigsäure-morpholid | | 15 mg |
| Suppositoriengrundmasse | ad | 2 g |

Beispiel 36:

Tabletten, enthaltend 30 mg Wirkstoff pro Tablette

| | pro Tablette |
|---|---|
| (4-(Methoxybenzoyl)-piperazin-1-yl)-essigsäure-morpholid | 30 mg |
| Lactat (feingemahlen) | 5 mg |
| Maisstärke (weiß) | 150 mg |
| Milchzucker | 60 mg |
| Mikrokristalline Cellulose | 50 mg |
| Polyvinylpyrrolidon | 20 mg |
| Magnesiumstearat | 2 mg |
| Natriumcarboximethylstärke | 25 mg |
| | 342 mg |

Beispiel 37:

Dragees, enthaltend einen erfindungsgemäßen Wirkstoff und einen anderen therapeutisch wirksamen Stoff

| | |
|---|---|
| (4-(Methoxybenzoyl)-piperazin-1-yl)-essigsäure-morpholid | 30 mg |
| Propranolol | 40 mg |
| Milchzucker | 90 mg |
| Maisstärke | 90 mg |
| Sec. Calciumphosphat | 34 mg |
| Lösliche Stärke | 3 mg |
| Magnesiumstearat | 3 mg |
| Kolloidale Kieselsäure | 4 mg |
| | 294 mg |

Beispiel 38:

Dragees, enthaltend einen erfindungsgemäßen Wirkstoff und einen anderen therapeutisch wirksamen Stoff

| | |
|---|---|
| (4-(Methoxybenzoyl)-piperazin-1-yl)-essigsäure-morpholid | 25 mg |
| Molsidomin | 5 mg |
| Milchzucker | 60 mg |
| Maisstärke | 90 mg |
| Sec. Calciumphosphat | 30 mg |
| Lösliche Stärke | 3 mg |
| Magnesiumstearat | 3 mg |
| Kolloidale Kieselsäure | 4 mg |
| | 220 mg |

Beispiel 39:

Kapseln, enthaltend einen erfindungsgemäßen Wirkstoff und einen anderen therapeutisch wirksamen Stoff.

| | |
|---|---|
| (4-(Methoxybenzoyl)-piperazin-1-yl)-essigsäure-morpholid | 20 mg |
| Prazosin | 5 mg |
| Maisstärke | 185 mg |
| | 210 mg |

Patentansprüche

1. Substituierte Piperazin-1-yl-essigsäure-amide der Formel I

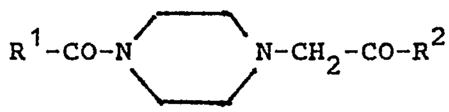

(I)

worin
$R^1$ Phenyl, ein-, zwei- oder dreifach, unabhängig voneinander durch Alkyl($C_1$-$C_4$), -O-Alkyl($C_1$-$C_4$), -CO-O-Alkyl($C_1$-$C_4$), $-SCH_3$, $-NH_2$, -NH-Alkyl($C_1$-$C_3$), $-N(Alkyl)_2$($C_1$-$C_2$ in jedem Alkylrest), -F, -Cl, -Br, -I, -OH oder -SH substituiertes Phenyl, Pyridyl, Thienyl, Furyl, Chlorphenoxymethyl, Amino, Alkylamino mit 1 bis 5 C-Atomen, Phenylamino, im Phenylkern durch -Cl, -Br, $-CH_3$ oder $-OCH_3$ substituiertes Phenylamino oder Alkoxy mit 1 bis 4 C-Atomen,
$R^2$ Amino, Alkylamino mit 1 bis 4 C-Atomen, Dialkylamino mit 1 bis 4 C-Atomen in jedem Alkylrest, Morpholino, 4-Methyl-piperazinyl-(1), Pyrrolidinyl-(1), Piperidino, Cyclohexamethylenimino, Diethanolamino, Dipropanolamino, $-NH(CH_2)_n-R^3$, $-NH(CH_2)_m-R^4$ oder 2-($R^3$-carbonyl)pyrrolin-1-yl,
$R^3$ Hydroxy, Alkoxy mit 1 bis 4 C-Atomen, Amino, Alkylamino mit 1 bis 4 C-Atomen, Dialkylamino mit 1 bis 4 C-Atomen in jedem Alkylrest,
$R^4$ Phenyl, Methoxyphenyl, Methylphenyl, Dimethoxyphenyl, Dimethylphenyl, Pyridyl,
n 2 oder 3,
m 1 oder 2
bedeuten sowie ihre Säureadditionsverbindungen.

2. Substituierte Piperazin-1-yl-essigsäure-amide nach Anspruch 1, dadurch gekennzeichnet, daß
$R^1$ Phenyl, Methoxyphenyl, Chlorphenyl, Bromphenyl, Fluorphenyl, Alkyl($C_1$-$C_4$)-phenyl, 4-Chlorphenoxymethyl, Dimethoxyphenyl, Dichlorophenyl, Methoxycarbonyl-phenyl, Acetoxymethoxy-phenyl, Alkoxy($C_1$-$C_2$), Amino, Chlorphenylamino, Trimethoxyphenyl, Thienyl, Pyridyl, Furyl bedeutet.

3. Substituierte Piperazin-1-yl-essigsäure-amide nach Anspruch 1 und/oder 2, dadurch gekennzeichnet, daß $R^2$ Amino, Alkyl($C_1$-$C_4$)-amino, Di-alkyl($C_1$-$C_4$)-amino, Morpholino, 4-Methyl-piperazinyl-(1), Pyrrolidinyl-(1), Piperidino, Diethanolamino, $-NH(CH_2)_n-R^3$, $-NH(CH_2)_m-R^4$ oder 2-($R^3$-Carbonyl)-pyrrolidin-1-yl bedeutet.

4. Substituiertes Piperazin-1-yl-essigsäureamid nach einem oder mehreren der Ansprüche 1 bis 3, dadurch gekennzeichnet, daß $R^3$ Hydroxy, Alkoxy mit 1 oder 2 C-Atomen, Amino, Alkylamino mit 1 oder 2 C-Atomen, Dialkylamino mit 1 oder 2 C-Atomen in jedem Alkylrest bedeutet.

5. Substituiertes Piperazin-1-yl-essigsäureamid nach einem oder mehreren der Ansprüche 1 bis 4, dadurch gekennzeichnet, daß $R^4$ Phenyl, Methoxyphenyl oder Dimethoxyphenyl bedeutet.

6. [4-(4-Methoxybenzoyl)-piperazin-1-yl]-essigsäure-morpholid oder ein pharmakologisch annehmbares Säureadditionssalz davon.

7. Verfahren zur Herstellung der substituierten Piperazin-1-yl-essigsäure-amide der Formel I und ihrer Säureadditionssalze eines oder mehrerer der Ansprüche 1 bis 6, dadurch gekennzeichnet, daß

a) ein Piperazin-1-yl-essigsäure-amid der Formel II

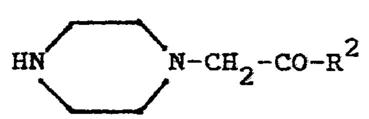

(II)

mit einem Acylierungsmittel III umgesetzt wird, das den Acylrest $R^1$-CO- einführt, oder daß

b) ein Piperazid der Formel IV mit einer Verbindung der Formel V

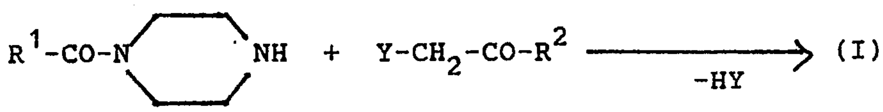


(IV) (V)

umgesetzt wird, wobei Y Halogen, $-OSO_2CH_3$, $-OSO_2$Phenyl oder -O-Tosyl bedeutet, oder daß

c) ein Piperazin-1-yl-essigsäure-derivat der Formel VI mit einem Amin der Formel VII

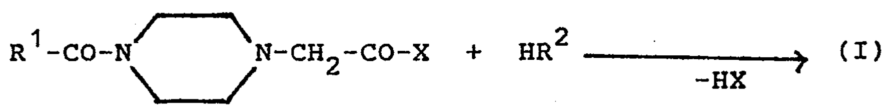


(VI) (VII)

umgesetzt wird, wobei X Halogen, $-O-CO-R^1$, -O-Alkyl, -OH bedeutet und ein gegebenenfalls anfallendes Säure-additionssalz in an sich bekannter Weise in die freie Verbindung der Formel I überführt wird, bzw. eine in freier Form anfallende Verbindung I gewünschtenfalls in an sich bekannter Weise in ein Säureadditionssalz überführt wird.

8. Verwendung der substituierten Piperazin-1-yl-essigsäureamide der Ansprüche 1 bis 6 als pharmakologische Wirkstoffe, insbesondere zur Bekämpfung und Vorbeugung der cerebralen Insuffizienz oder bei der Verbesserung der intellektuellen Leistungsfähigkeit.

9. Verfahren zur Behandlung von Menschen zur Bekämpfung und Vorbeugung der cerebralen Insuffizienz oder bei der Verbesserung der intellektuellen Leistungsfähigkeit, dadurch gekennzeichnet, daß eine wirksame Dosis einer Verbindung der Ansprüche 1 bis 6 verabreicht wird.

10. Pharmazeutisches Präparat, dadurch gekennzeichnet, daß es als Wirkstoff eine Verbindung der Ansprüche 1 bis 6 oder ein pharmazeutisch annehmbares Säureadditionssalz davon, zusammen mit einem pharmazeutisch annehmbaren Trägerstoff und gegebenenfalls pharmazeutisch annehmbaren Zusatzstoffen und gegebenenfalls noch eine oder mehrere andere pharmakologische Wirkstoffe enthält.

P A T E N T A N S P R Ü C H E
für das Benennungsland Österreich

1. Verfahren zur Herstellung von substituierten Piperazin-1-yl-essigsäure-amiden der Formel I

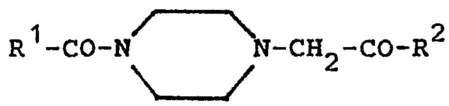

(I)

worin

$R^1$ Phenyl, ein-, zwei- oder dreifach, unabhängig voneinander durch Alkyl($C_1$-$C_4$), -O-Alkyl($C_1$-$C_4$), -CO-O-Alkyl-($C_1$-$C_4$), $-SCH_3$, $-NH_2$, -NH-Alkyl($C_1$-$C_3$), -N(Alkyl)$_2$($C_1$-$C_2$ in jedem Alkylrest), -F, -Cl, -Br, -I, -OH oder -SH substituiertes Phenyl, Pyridyl, Thienyl, Furyl, Chlorphenoxymethyl, Amino, Alkylamino mit 1 bis 5 C-Atomen, Phenylamino, im Phenylkern durch -Cl, -Br, $-CH_3$ oder $-OCH_3$ substituiertes Phenylamino oder Alkoxy mit 1 bis 4 C-Atomen,

$R^2$ Amino, Alkylamino mit 1 bis 4 C-Atomen, Dialkylamino mit 1 bis 4 C-Atomen in jedem Alkylrest, Morpholino, 4-Methyl-piperazinyl-(1), Pyrrolidinyl-(1), Piperidino, Cyclohexamethylenimino, Diethanolamino, Dipropanolamino, $-NH(CH_2)_n-R^3$, $-NH(CH_2)_m-R^4$ oder 2-($R^3$-Carbonyl)pyrrolin-1-yl,

$R^3$ Hydroxy, Alkoxy mit 1 bis 4 C-Atomen, Amino, Alkylamino mit 1 bis 4 C-Atomen, Dialkylamino mit 1 bis 4 C-Atomen in jedem Alkylrest,

$R^4$ Phenyl, Methoxyphenyl, Methylphenyl, Dimethoxyphenyl, Dimethylphenyl, Pyridyl,

n 2 oder 3,

m 1 oder 2

bedeuten sowie ihre Säureadditionsverbindungen, dadurch gekennzeichnet, daß

a)

ein Piperazin-1-yl-essigsäure-amid der Formel II

0123977
Ref. 3274
Dr.Eu/Ll

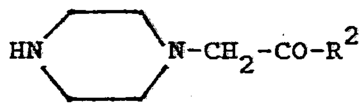
(II)

mit einem Acylierungsmittel III umgesetzt wird, das den Acylrest $R^1$-CO- einführt, oder daß

b)

ein Piperazid der Formel IV mit einer Verbindung der Formel V

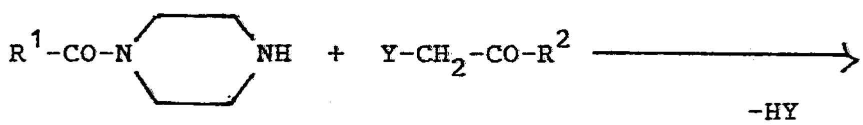

(I)

(IV) (V)

umgesetzt wird, wobei Y Halogen, $-OSO_2CH_3$, $-OSO_2$Phenyl oder -O-Tosyl bedeutet, oder daß

c)

ein Piperazin-1-yl-essigsäure-derivat der Formel VI mit einem Amin der Formel VII

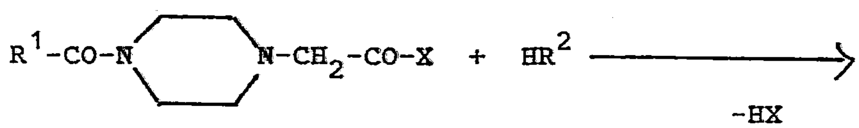

(I)

(VI) (VII)

umgesetzt wird, wobei X Halogen, -O-CO-$R^1$, -O-Alkyl, -OH, bedeutet und ein gegebenenfalls anfallendes Säureadditionssalz in an sich bekannter Weise in die freie Verbindung der Formel I überführt wird, bzw. eine in freier Form anfallende Verbindung I gewünschtenfalls in an sich bekannter Weise in ein Säureadditionssalz überführt wird.

2. Verfahren nach Anspruch 1, dadurch gekennzeichnet, daß die Umsetzung in einem inerten Lösungsmittel oder Lösungsmittelgemisch durchgeführt wird.

3. Verfahren nach Anspruch 1 und/oder 2, dadurch gekennzeichnet, daß die Umsetzung bei Temperaturen von $-10^{\circ}C$ bis $25^{\circ}C$ durchgeführt wird.

4. Verfahren nach einem oder mehreren der Ansprüche 1 bis 3, dadurch gekennzeichnet, daß die Ausgangsverbindungen so ausgewählt werden, daß Verbindungen der Formel I oder Säureadditionssalze davon entstehen, wobei $R^1$ Phenyl, Methoxyphenyl, Chlorphenyl, Bromphenyl, Fluorphenyl, Alkyl($C_1$-$C_4$)-phenyl, 4-Chlorphenoxymethyl, Dimethoxyphenyl, Dichlorophenyl, Methoxycarbonyl-phenyl, Acetoxy-methoxy-phenyl, Alkoxy($C_1$-$C_2$), Amino, Chlorphenylamino, Trimethoxyphenyl, Thienyl, Pyridyl, Furyl bedeutet.

5. Verfahren nach einem oder mehreren der Ansprüche 1 bis 4, dadurch gekennzeichnet, daß die Ausgangsprodukte so ausgewählt werden, daß Verbindungen der Formel I oder Säureadditionssalze davon entstehen, wobei $R^2$ Amino, Alkyl($C_1$-$C_4$)-amino, Di-alkyl($C_1$-$C_4$)-amino, Morpholino, 4-Methyl-piperazinyl-(1), Pyrrolidinyl-(1), Piperidino, Diethanolamino, $-NH(CH_2)_n-R^3$, $-NH(CH_2)_m-R^4$ oder 2-($R^3$-Carbonyl)-pyrrolidin-1-yl bedeutet.

6.Verfahren nach einem oder mehreren der Ansprüche 1 bis 5, dadurch gekennzeichnet, daß die Ausgangsprodukte so ausgewählt werden, daß Verbindungen der Formel I oder Säureadditionssalze davon entstehen, wobei $R^3$ Hydroxy, Alkoxy mit 1 oder 2 C-Atomen, Amino, Alkylamino mit 1 oder 2 C-Atomen, Dialkylamino mit 1 oder 2 C-Atomen in jedem Alkylrest bedeutet.

7. Verfahren nach einem oder mehreren der Ansprüche 1 bis 6, dadurch gekennzeichnet, daß die Ausgangsverbindungen so ausgewählt werden, daß Verbindungen der Formel I oder

Säureadditionssalze davon entstehen, wobei $R^4$ Phenyl, Methoxyphenyl oder Dimethoxyphenyl bedeutet.

8. Verfahren nach einem oder mehreren der Ansprüche 1 bis 7, dadurch gekennzeichnet, daß die Ausgangsverbindungen so ausgewählt werden, daß die Verbindung (4-(4-Methoxybenzoyl)-piperazin-1-yl)-essigsäure-morpholid oder ein pharmakologisch annehmbares Säureadditionssalz davon entsteht.

9. Verwendung der nach den Ansprüchen 1 bis 8 erhältlichen substituierten Piperazin-1-yl-essigsäure-amide oder ihrer pharmakologisch annehmbbaren Säureadditionssalze als pharmakologische Wirkstoffe, insbesondere zur Bekämpfung und Vorbeugung der cerebralen Insuffizienz oder bei der Verbesserung der intellektuellen Leistungsfähigkeit.

10. Verfahren zur Herstellung pharmazeutischer Präparate auf nicht chemischem Wege, dadurch gekennzeichnet, daß als Wirkstoff eine der in Anspruch 1 angegebene Verbindung der Formel I oder ein pharmakologisch annehmbares Säureadditionssalz davon in an sich bekannter Weise zusammen mit einem pharmazeutisch annehmbaren Trägerstoff und gegebenenfalls pharmazeutisch annehmbaren Zusatzstoffen und gegebenenfalls noch eine oder mehrere andere pharmakologische Wirkstoffe gemischt wird.

0123977

Nummer der Anmeldung

Europäisches Patentamt

# EUROPÄISCHER RECHERCHENBERICHT

EP 84 10 4042

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| X | FR-A-2 081 564 (DELALANDE)<br>* Seite 1, Zeilen 1-21; Beispiel 3; Seite 5, Nr. 69189; Seite 6, Nr. 69160; Seiten 8-10 *<br>--- | 1-10 | C 07 D 295/18<br>C 07 D 295/20<br>C 07 D 307/68<br>C 07 D 213/82<br>C 07 D 207/16<br>C 07 D 409/12<br>A 61 K 31/495 |
| X | US-A-4 247 549 (OHNMACHT)<br>* Spalte 1, Zeilen 1-33; Spalte 20, Zeilen 66-68; Spalte 22, Zeile 63; Spalte 23, Zeile 63; Spalte 27, Zeile 55 *<br>--- | 1-10 | |
| X | FR-A-2 113 942 (LABORATOIRES BIOSEDRA)<br>* Seite 1, Zeilen 1-19; Tafel I: Verbindungen 4,7; Seiten 5,6 *<br>--- | 1-10 | |
| A | EP-A-0 064 878 (OTSUKA PHARMACEUTICAL)<br>* Ansprüche *<br>----- | 1-10 | **RECHERCHIERTE SACHGEBIETE (Int. Cl. 3)**<br>C 07 D 295/00<br>C 07 D 207/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 27-07-1984 | PAUWELS G.R.A. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82